# EUROPEAN PATENT APPLICATION

(11) **EP 4 501 863 A1**
(43) Date of publication of application: **05.02.2025**
(21) Application number: 24190799.7
(22) Date of filing: 25.07.2024
(51) Int. Cl.: C02F 1/00, C02F 1/28, C02F 1/44, G01N 21/49

(54) **WATER DISPENSING APPARATUS**

(30) Priority: 02.08.2023 KR 20230101179
(71) Applicant: LG Electronics Inc., Yeongdeungpo-gu Seoul 07336 (KR)
(72) Inventor: Han, Dongkoo, 08592 Seoul (KR)
(74) Representative: Ter Meer Steinmeister & Partner

(57) **Abstract**

A water dispensing apparatus including a housing forming an outer appearance; a water supply flow path configured to flow raw water supplied from an external water supply source into the housing; a filter disposed inside the housing to generate purified water by filtering the raw water supplied to the water supply flow path; a purified water flow path configured to flow purified water that has passed through the filter toward a water outlet part provided outside the housing; a detection means installed in at least one of the water supply flow path or the purified water flow path to measure water quality data including turbidity of flowing water; and a control means configured to receive water quality data of raw water or purified water detected by the detection means.

## Description

### The Background

The present disclosure relates to a water dispensing apparatus that may detect the turbidity of purified water and determine whether it is suitable for drinking.

A water dispensing apparatus is an apparatus that supplies water and is capable of dispensing a desired amount of water at a desired temperature according to a user's operation. Such water dispensing apparatus may be applied to various fields, but are typically applied to refrigerators and water purifiers. In particular, water dispensing apparatus provided in refrigerators and water purifiers are configured to have a function of supplying a preset amount of water according to a user's operation. Recently, water dispensing apparatus have been developed that may supply not only purified water but also cold water and hot water.

For example, a water purifier is configured to be connected to a water supply source such as a tap so that raw water receives, to use a filter so that suspended matter and harmful components contained in the raw water are removed, and to purify and dispense as much water as desired according to the user's operation. Various water purifiers such as these are being released that can not only purify water, but also heat or cool the purified water and supply it as cold or hot water. In addition, recently, water purifiers that are small in size and may be installed in various installation environments have been developed.

If a water dispensing apparatus is used for a long time, microorganisms in pipes, valves, and water outlet parts may multiply or become contaminated, and according to the replacement cycle of the filter, suspended matter or harmful components contained in the raw water may not be removed. Therefore, it is important for a water dispensing apparatus to accurately measure water quality and manage it hygienically, and must also manage purified water quality performance.

According to Korea Patent Publication No. 10-2022-0105771 as the prior document, a method for recommending a suitable filter and water purifier to a customer is disclosed.

In other words, a method for recommending a water purifier to customers is disclosed and the method includes generating location information where the water purifier is to be installed, building age data using publicly accessible information, and estimated pollution level data of raw water provided to the buildings, if the estimated pollution level data is in the first range, recommending a reverse osmosis type water purifier, and if the estimated pollution level data is in a second range that is different from the first range, recommending a non-reverse osmosis type water purifier.

Specifically, the first range is a data range corresponding to a case where the type of raw water is groundwater, the age data of the building is outside the preset range, or the water quality data indicates an area at risk of water pollution, and the second range is a data range corresponding to a case where the type of raw water is tap water, the age data of the building is within the preset range, and the water quality data indicates that it is not an area at risk of water pollution.

Recommending a water purifier involves determining a filter suitable for the predicted pollution, and the first filter and the second filter may be distinguished and recommended to the user according to the degree of pollution.

The second filter is a filter with excellent water purification performance, and the first filter is a filter with lower water purification performance than the second filter, and for example, the second filter includes a reverse osmosis filter, and the first filter is a nanotrap filter, a hollow fiber membrane filter, or the like.

However, when installing a filter and water purifier as described above, it is bound to be difficult to check in real time whether the quality of the drinking water discharged through the water outlet nozzle is drinkable, and it is bound to be difficult to immediately respond to situations where the quality of raw water changes due to climate change or environmental changes. In particular, there is a problem that in situations where rusty or muddy water flows in, the user is unaware of this and has no choice but to drink the water, which has turbidity levels that are unsuitable for drinking even if it has passed through the filter.

### The Summary

It is an object of the present disclosure to propose a water dispensing apparatus that is configured to sense the real-time turbidity of raw water or purified water, and/or to detect and respond early when the quality of raw water deteriorates due to the inflow of rusty water or other reasons.

It is an object of the present disclosure to propose a water dispensing apparatus that is configured to measure and/or analyze the water quality at the final stage of raw water flowing in from water sources, including water supply pipes, and water discharged through a water outlet nozzle.

It is an object of the present disclosure to propose a water dispensing apparatus in which sensors are miniaturized that may measure turbidity which is one of the criteria for determining whether the water is suitable for drinking and/or Total Dissolved Solids (TDS) levels which are commonly used as water quality characteristics, and/or in which sensors with improved sensing precision are applied to measure the quality of raw water and purified water more quickly and accurately.

It is an object of the present disclosure proposes a water dispensing apparatus that may be easily applied to products that are already installed or have been commercialized.

It is an object of the present disclosure to propose a water dispensing apparatus that may manage and visualize differences in water quality measurements between raw water and purified water.

It is an object of the present disclosure to propose a water dispensing apparatus that may reduce material costs by modularizing a plurality of sensors and/or may configure the product compactly.

It is an object of the present disclosure to propose a water dispensing apparatus that is configured to easily determine and/or manage the pollution level of raw water and purified water.

A water dispensing apparatus according to an aspect of the present disclosure includes a housing forming an outer appearance; a water supply flow path configured to flow raw water supplied from an external water supply source into the housing; a filter disposed inside the housing to generate purified water by filtering the raw water supplied to the water supply flow path; a purified water flow path configured to flow purified water that has passed through the filter toward a water outlet part provided outside the housing; a detection means installed in at least one of the water supply flow path or the purified water flow path to measure water quality data including turbidity of flowing water; and a control means configured to receive water quality data of raw water or purified water detected by the detection means.

A water dispensing apparatus according to another aspect of the present disclosure includes: a filter configured to generate purified water by filtering the water; a water supply flow path for supplying water from an external water supply source to the filter; a purified water flow path for supplying the purified water to or toward a water outlet part; a detection means installed in at least one of the water supply flow path and the purified water flow path and configured to measure water quality data (e.g. turbidity of water, content or concentration or amount of ions, chlorine or total dissolved solids), in particular of/in the flowing water; and a control means configured to receive water quality data (e.g. of raw water and/or purified water) detected by the detection means.

The water dispensing apparatus according to any one of these aspects may include one or more of the following features:
The water dispensing apparatus may also be denoted as "water dispensing system".
The water dispensing apparatus may include a housing, in which the filter is disposed. The housing may form an outer appearance. The water outlet part may be provided outside the housing. The water outlet part may include a water tap, water plug, spigot or water connection, e.g. a water connection for home appliances.
Water from the external water supply source (and before having passed through the filter) and/or water that has not passed the filter may be denoted as "raw water". Water having passed through the filter may be denoted as "purified water". The water supply flow path may also be denoted as "raw water flow path".
The water dispensing apparatus may be connected between the water supply flow path and the purified water flow path.

The detection means may be configured to measure water quality data (e.g. turbidity of water) of water flowing in the water supply flow path and/or the purified water flow path.

The detection means may include at least one of a turbidity sensor, an ion sensor, a pH sensor, a chlorine sensor, a Total Dissolved Solids (TDS) sensor, and a Biochemical Oxygen Demand (BOD) sensor and may measure at least one of the turbidity, pH, residual chlorine, Total Dissolved Solids (TDS), and dissolved oxygen of incoming water. The detection means may include at least one of a turbidity sensor for measuring turbidity, an ion sensor for measuring pH or an amount of ions, a chlorine sensor for measuring an amount of residual chlorine, a Total Dissolved Solids (TDS) sensor for measuring an amount of Total Dissolved Solids (TDS), and a Biochemical Oxygen Demand (BOD) sensor for measuring an amount of dissolved oxygen.

The detection means may include a turbidity sensor. The turbidity sensor may be configured to irradiate light to a portion of the raw water or the purified water and to sense turbidity based on a pattern of scattered light received. The turbidity sensor may be configured to irradiate light to the water, in particular to (only) a portion of the water, receive a pattern of scatter light and to sense turbidity based on the pattern of scattered light.

The turbidity sensor may include a measuring container with a light incident part to input laser light therethrough, a light scattering space formed inside the measuring container, and a light emitting part to receive the pattern of scattered light generated in the light scattering space for measuring microscopic turbidity or pollution by bacteria or microorganisms.

The measuring container may have an overall cylindrical shape made of at least metal, glass, synthetic resin, and combinations thereof, with scattering protrusions or scattering layers formed on the inner surface. The measuring container may have a cylindrical shape and/or may be made of at least one of metal, glass, synthetic resin, and combinations thereof and/or may have scattering protrusions or scattering layers formed on an inner surface thereof.

The detection means may include at least one of a turbidity sensor, an ion sensor, a chlorine sensor, and a Total Dissolved Solids (TDS) sensor.

The water quality data may include at least one of turbidity, (amount of) residual chlorine, (amount of) Total Dissolved Solids (TDS), and (amount of) dissolved oxygen.

The detection means may include a first turbidity sensor installed in the purified water flow path to sense turbidity of purified water, i.e. the water flowing in the purified water flow path.

The detection means may include a first TDS sensor installed in the purified water flow path to sense the amount of total dissolved solids (TDS) contained in purified water, i.e. the water flowing through the purified water flow path.

The detection means may include a second turbidity sensor installed in the water supply flow path or raw water flow path to sense turbidity of (raw water) flowing therethrough.

The detection means may include a second TDS sensor installed in the water supply flow path or raw water flow path to sense the amount of total dissolved solids (TDS) contained in the (raw) water flowing therethrough.

The control means may perform feedback control based on water quality data measured by the detection means. The control means may be configured to control an output part. The output part may be part of the water dispensing apparatus or may be a separate unit connected by wire or wirelessly (e.g. by Bluetooth, WLAN, etc.) to the water dispensing apparatus, in particular to the control means of the water dispensing apparatus. In the latter case, the output part may be a electronic mobile device, e.g. a smartphone or tablet, or a display unit of a home appliance, e.g. a refrigerator.

The output part may be configured or controlled to display information on water quality based on or corresponding to the measured water quality data, e.g. to display information on abnormal water quality when the measured water quality data is outside a preset normal range.

The water dispensing apparatus may further include when the water quality data of the (raw) water or purified water is outside a preset normal range, an output part configured to display information on abnormal water quality of the (raw) water or purified water.

The detection means may be configured to detect the turbidity and TDS amount of (raw) water or purified water, and even if the detected TDS amount is within the normal range, if the detected turbidity is outside the normal range, the output part may be configured or controlled to display information on water quality abnormalities of raw water or purified water on the output unit.

A water outlet valve may be disposed between the filter and the water outlet part to control the flow of purified water flowing toward the water outlet part. The water outlet valve and/or the detection means may be installed in a purified water flow path connecting the filter and the water outlet valve.

An adjustment means may be disposed in the water supply path and/or between the water supply source and the filter. The adjustment means may be configured to adjust at least one of the pressure, flow velocity, and flow rate of the water flowing into the filter, in particular to lower at least one of the pressure, flow velocity, and flow rate of the water flowing into the filter.

An adjustment means disposed between the water supply source and the filter to adjust to be lower at least one of the pressure, flow velocity, or flow rate of the raw water flowing into the filter may be installed in the raw water flow path.

The detection means may be installed in a water flow path connecting the adjustment means and the filter and/or between the adjustment means and the filter. The detection means may be installed in a (raw) water flow path connecting the adjustment means and the filter.

The water outlet part may be separated from the housing and/or installed in a location spaced apart from the housing and/or connected to the housing by the purified water flow path.

The detection means may be installed on a water supply flow path (or raw water flow path) or on a purified water flow path. The detection means may be installed exposed to the outside of the housing or accommodated inside the housing. The detection means may be installed on a water supply flow path (or raw water flow path) or on a purified water flow path exposed to the outside of the housing or accommodated inside the housing. The water outlet part may be part of the water dispensing apparatus or a separate entity connected to the water dispensing apparatus. The water outlet part may be separated from the housing and installed at a location spaced apart from the housing, and the detection means may be installed on a raw water flow path or purified water flow path accommodated inside the housing.

The water outlet part may be provided integrally with the housing to be exposed to the outside of the housing. The detection means may connect the housing and the water outlet part. The detection means may be installed on a water supply flow path (or raw water flow path) or on a purified water flow path accommodated inside the housing.

The detection means may further include an outer cover forming an installation space on the inside thereof.

The water dispensing apparatus may further include a first turbidity sensor. The first turbidity sensor may be disposed between the filter and the water outlet part. The first turbidity sensor may be installed in the installation space.

The purified water flow path may include a purified water input pipe. The purified water input pipe may be disposed below or in front of (in flow direction of water) the first turbidity sensor. The purified water input pipe may be configured to supply purified water supplied from the filter side to the first turbidity sensor. That is, the purified water input pipe may connect the filter to the first turbidity sensor. The purified water flow path may include a purified water output pipe. The purified water output pipe may be disposed above or behind (in flow direction of water) the first turbidity sensor. The purified water output pipe may be configured to supply purified water passing through the first turbidity sensor to the water outlet part. That is, the purified water output pipe may connect the first turbidity sensor to the water outlet part.

The detection means may include a first TDS sensor. The first TDS sensor may be disposed between the first turbidity sensor and the water outlet part and/or on the purified water output pipe to measure the TDS amount of purified water having passed through the first turbidity sensor. The first TDS sensor may be disposed between the first turbidity sensor and the purified water output pipe to measure the TDS amount of purified water passing through the first turbidity sensor.

The purified water flow path may further include the purified water connection pipe connecting an outlet end of the first turbidity sensor and an inlet end of the first TDS sensor.

The detection means may further include a second turbidity sensor. The second turbidity sensor may be installed between the water supply source and the filter. The second turbidity sensor may be installed in the installation space.

The water supply flow path or raw water flow path may include a (raw) water input pipe. The (raw) water input pipe may be disposed below or in front of (in flow direction of water) the second turbidity sensor. The (raw) water input pipe may be configured to supply (raw) water supplied from the water supply source to the second turbidity sensor. That is, the (raw) water input pipe may connect the water supply source to the second turbidity sensor. The water supply flow path or raw water flow path may include a (raw) water output pipe. The (raw) water output pipe may be disposed above or behind (in flow direction of water) the second turbidity sensor. The (raw) water output pipe may be configured to supply (raw) water passing through the second turbidity sensor to the filter. That is, the (raw) water output pipe may connect the second turbidity sensor to the filter.

The detection means may include a second TDS sensor. The second TDS sensor may be disposed between the external water supply source and the second turbidity sensor and/or on the water supply flow path (or raw water flow path) to sense or measure the amount of total dissolved solids (TDS) contained in the (raw) water flowing therethrough

The water dispensing apparatus may further include a pressure reducing valve installed in the installation space. The pressure reducing valve may be disposed between the second turbidity sensor and the (raw) water input pipe to adjust the pressure of the (raw) water flowing into the (raw) water input pipe and then supply the (raw) water to the second turbidity sensor.

The control means may be installed in the installation space and/or disposed on an upper part of the purified water output pipe.

The control means may be connected to a separate output part and transmit the input or measured water quality data to the output part.

### BRIEF DESCRIPTION OF THE DRAWINGS

FIG. 1 is a conceptual diagram illustrating water quality sensing of a water dispensing apparatus according to an embodiment of the present disclosure.
FIG. 2 is a front view illustrating a detection means, which is a main component of a water dispensing apparatus according to an embodiment of the present disclosure.
FIG. 3 is an exploded perspective view illustrating a detection means, which is a main component of a water dispensing apparatus according to an embodiment of the present disclosure.
FIGS. 4 to 6 are water piping diagrams illustrating water dispensing apparatus according to various embodiments of the present disclosure.
FIG. 7 is a table comparing the TDS amount and turbidity (NTU) of rusty water of various concentrations.
FIG. 8 is a graph comparing the amount of TDS and turbidity (NTU) of rusty water of various concentrations.
FIGS. 9 to 10 are diagrams conceptually illustrating a monitoring system for a water dispensing apparatus according to the present disclosure.
FIG. 11 is a water piping diagram illustrating a water dispensing apparatus according to another embodiment of the present disclosure.
FIGS. 12 to 15 are diagrams illustrating various embodiments of filters applied to water dispensing of the present disclosure.

### DETAILED DESCRIPTION

Hereinafter, embodiments of the present disclosure will be described in detail with reference to the attached drawings. However, the present disclosure is not limited to these embodiments and may of course be modified into various forms.

In the drawings, parts not related to the description are omitted in order to clearly and briefly explain the present disclosure, and identical or extremely similar parts are denoted by the same drawing reference numerals throughout the specification.

Additionally, in this specification, terms such as first and second may be used to describe various elements, but these elements are not limited by these terms. These terms are only used to distinguish one element from another.

The water dispensing apparatus of the present disclosure has the function of measuring and monitoring water flowing into the filter and/or purified water, cold water, and hot water discharged through the filter.

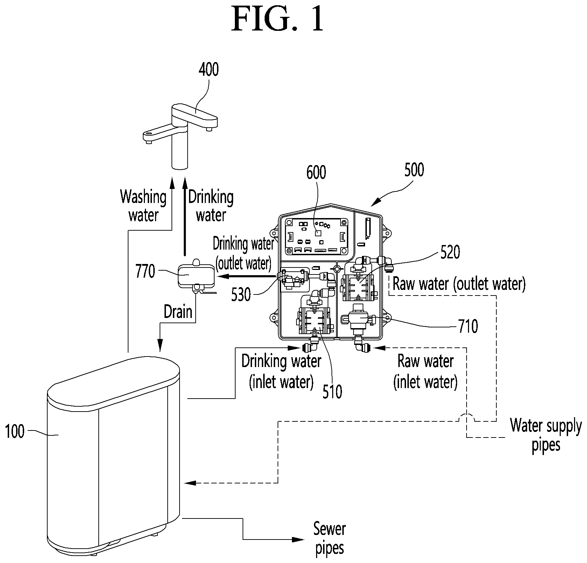

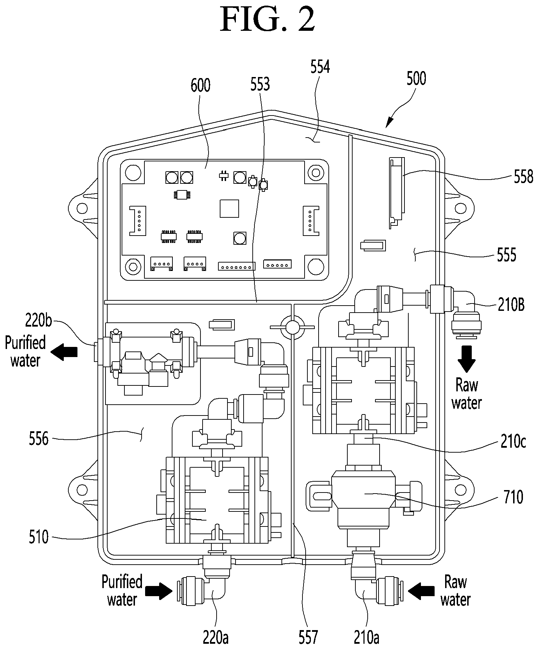

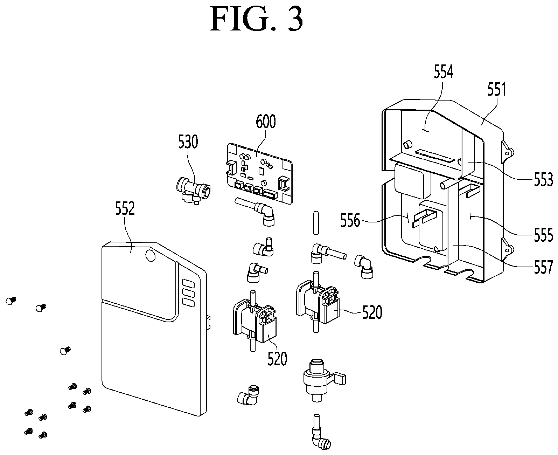

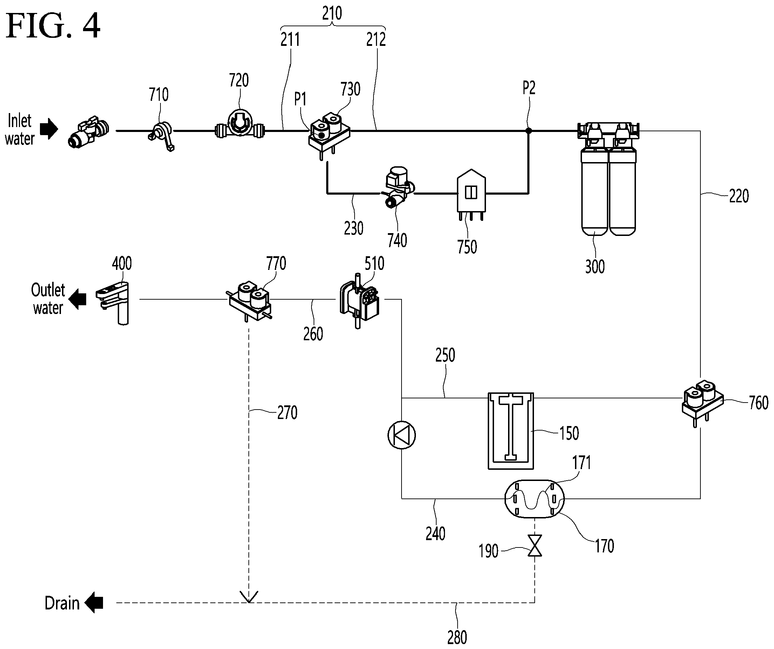

FIG. 1 is a conceptual diagram illustrating water quality sensing of a water dispensing apparatus according to an embodiment of the present disclosure, FIG. 2 is a front view illustrating a detection means, which is a main component of a water dispensing apparatus according to an embodiment of the present disclosure, FIG. 3 is an exploded perspective view illustrating a detection means, which is a main component of a water dispensing apparatus according to an embodiment of the present disclosure, and FIGS. 4 to 6 are water piping diagrams illustrating water dispensing apparatus according to various embodiments of the present disclosure.

Referring to FIGS. 1 to 6, the water dispensing apparatus according to the present disclosure includes a housing 100 forming an outer appearance, a water supply flow path 210 through which raw water supplied from an external water supply source flows into the housing, a filter 300 disposed inside the housing 100 and generating purified water by filtering the raw water supplied to the water supply flow path 210, a purified water flow path 220 flowing the purified water that has passed through the filter 300 toward the water outlet part 400 provided outside of the housing 100, a detection means 500 installed at least one of the water supply flow path 210 or the purified water flow path 220 and measuring water quality data including turbidity of the flowing water, and a control means 600 that receives water quality data of raw water or purified water detected by the detection means 500.

In the following description, it should be noted that the purified water flow path 220 refers to all flow paths through which purified water passing the filter 300 has passed.

In particular, the hot water flow path 240, cold water flow path 250, and outlet water flow path 260, which will be described later, are flow paths through which purified water passing through the filter 300 flows, so they may be included in the scope of the purified water flow path 220.

The detection means 500 is installed in the water supply flow path 210 and may sense water quality information including turbidity of raw water flowing toward the filter 300.

In the above embodiment, the detection means 500 may be installed between the water supply source and the filter 300 based on the water flow direction.

The detection means 500 may be installed at the outlet end of the water supply source or the inlet end of the filter 300.

Alternatively or additionally, the detection means 500 is installed in the purified water flow path 220 and may sense water quality information including turbidity of purified water flowing toward the water outlet part 400 after passing through the filter 300.

In the above embodiment, the detection means 500 may be installed between the filter 300 and the water outlet part 400 based on the water flow direction.

The detection means 500 may be installed at the outlet end of the filter 300 or the inlet end of the water outlet part 400.

The detection means 500 may be installed in each of the water supply flow path 210 and the purified water flow path 220 to sense the water quality information including turbidity of the raw water flowing toward the filter 300 and the purified water flowing toward the water outlet part 400 after passing through the filter 300.

In the above embodiment, the detection means 500 may be installed between the water supply source and the filter 300, and between the filter 300 and the water outlet part 400, based on the direction of water flow.

For instance, the detection means 500 may be installed at the outlet end of the water supply source or the inlet end of the filter 300, and may be installed at the outlet end of the filter 300 or the inlet end of the water outlet part 400.

Meanwhile, the filter 300 is for purifying raw water supplied from a water supply source and filters out various impurities and harmful matter contained in the raw water. The filter 300 may be provided as one or more, and when provided in plural, filters having various functions may be combined. For example, the filter 300 may be provided in three pieces, including a pre-carbon filter, a post-carbon filter, and a membrane filter or hollow fiber membrane filter disposed between the pre-carbon filter and the post-carbon filter. Alternatively, the filter 300 may be composed of a pre-carbon filter and a UF composite filter.

The purified water purified by the filter 300 is stored in a storage tank or flows into the purified water flow path 220. In the case of the water storage type, purified water that has passed the filter 300 is stored in the storage tank. In the case of the direct water type, the water that has passed through the filter 300 flows directly to the water outlet part side through the purified water flow path 220.

The detection means 500 may include turbidity sensors 510 and 520 that irradiate light to a portion of the raw water or the purified water and sense turbidity based on the received scattered light pattern.

For example, the turbidity sensor 510 includes a measuring container in which a light incident part is formed on one side so that laser light may be input, a light scattering space is formed inside where the laser light may be multiple reflections or multiple scattering in multiple paths, and in which a light emitting part is formed on the other side to measure microscopic turbidity or pollution by bacteria or microorganisms using a speckle pattern generated in the light scattering space.

In addition, the measuring container has an overall cylindrical shape made of at least metal, glass, synthetic resin, and combinations thereof, with scattering protrusions or scattering layers formed on the inner surface.

In addition, the detection means 500 may include at least one of a turbidity sensor 510 and 520, an ion sensor, a chlorine sensor, a Total Dissolved Solids (TDS) sensor, and a Biochemical Oxygen Demand (BOD) sensor and may measure at least one of the turbidity, pH, residual chlorine, Total Dissolved Solids (TDS), and dissolved oxygen of incoming water.

According to the present disclosure, the detection means 500 may sense the turbidity of purified water. Also, the detection means 500 may sense the turbidity of raw water and/or purified water.

Referring to FIGS. 4 to 6, the detection means 500 may include a first turbidity sensor 510 that is installed in the purified water flow path 220 and senses the turbidity of purified water flowing through the purified water flow path 220.

The first turbidity sensor 510 may be installed in at least one of the purified water flow path 220 as well as the flow path through which purified water passing through the filter 300 flows.

In other words, the first turbidity sensor 510 may be installed in at least one of the purified water flow path 220, the hot water flow path 240, the cold water flow path 350, and the water outlet flow path 260.

The first turbidity sensor 510 may be installed together with valves, electrical components, or the like installed in the flow path through which purified water that has passed through the filter 300 flows.

In case that the first turbidity sensor 510 is installed in the hot water flow path 240, it may sense the turbidity of hot water flowing through the hot water flow path 240.

In case that the first turbidity sensor 510 is installed in the cold water flow path 250, it may sense the turbidity of cold water flowing through the cold water flow path 250.

In case that the first turbidity sensor 510 is installed in the water outlet flow path 260, it may sense the turbidity of water flowing through the water outlet flow path 260, e.g. the turbidity of purified water, hot water, or cold water flowing through the water outlet flow path 260.

When the first turbidity sensor 510 detects the turbidity of the purified water, the control means 600 may collect turbidity data of the purified water.

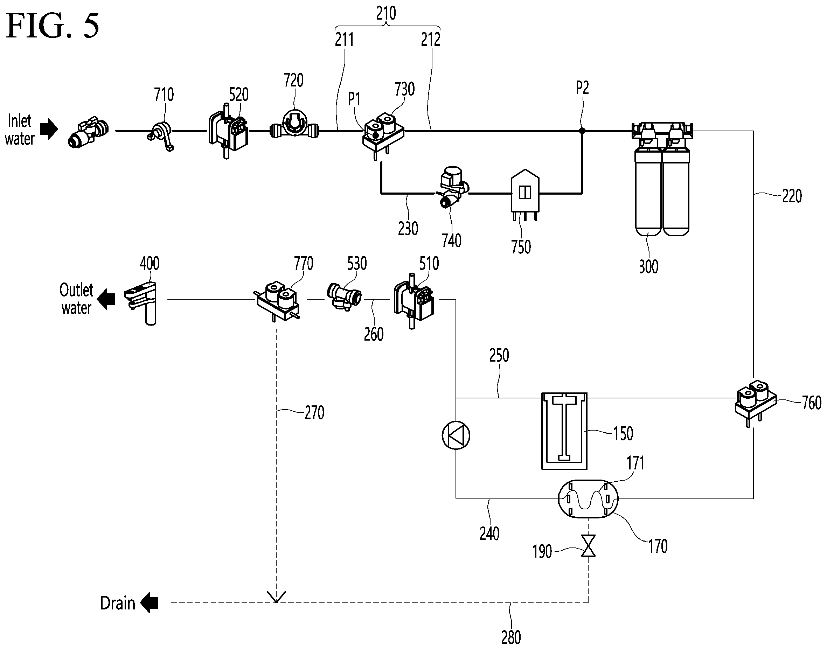

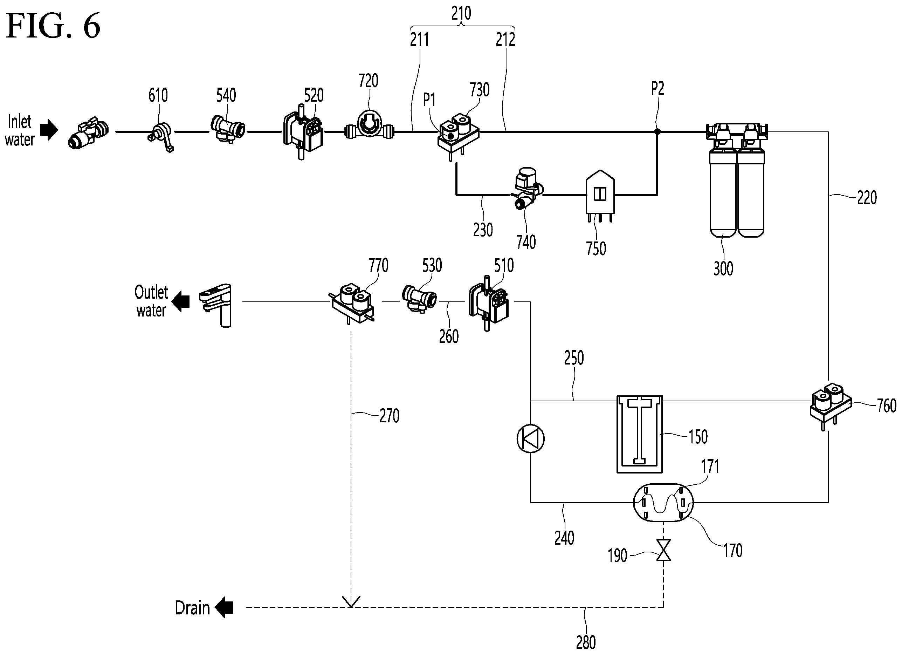

In addition, referring to FIGS. 5 and 6, the detection means 500 may include a first TDS sensor 530 installed in the purified water flow path 220 and sensing the amount of Total Dissolved Solids (TDS) contained in the purified water flowing through the purified water flow path 220.

The first TDS sensor 530 may be installed in at least one of the purified water flow path 220 as well as the flow path through which purified water passing through the filter 300 flows.

In other words, the first TDS sensor 530 may be installed in at least one of the purified water flow path 220, the hot water flow path 240, the cold water flow path 350, and the outlet water flow path 260.

The first TDS sensor 530 may be installed together with valves, electrical components, or the like installed in the flow path through which purified water passing through the filter 300 flows.

The first TDS sensor 530 is installed in the hot water flow path 240 and may sense the amount of total dissolved solids (TDS) contained in hot water flowing through the hot water flow path 240.

The first TDS sensor 530 is installed in the cold water flow path 250 and may sense the amount of total dissolved solids (TDS) contained in cold water flowing through the cold water flow path 250.

The first TDS sensor 530 is installed in the water outlet flow path 260 and may sense the amount of total dissolved solids (TDS) contained in purified water, hot water, or cold water flowing through the water outlet flow path 260.

When the first TDS sensor 530 detects the amount of total dissolved solids (TDS) contained in the purified water, the control means 600 collects the total dissolved solids (TDS) data of the purified water.

For instance, referring to FIG. 6, the detection means 500 may include a second turbidity sensor 520 that is installed in the raw water flow path 210 and senses the turbidity of the raw water flowing through the raw water flow path 210.

When the second turbidity sensor 520 detects the turbidity of the raw water, the control means 600 may collect turbidity data of the raw water.

The detection means 500 may include a second TDS sensor 540 installed in the raw water flow path 210 to sense the amount of total dissolved solids (TDS) contained in the raw water flowing through the raw water flow path 210.

When the second TDS sensor 540 detects the amount of total dissolved solids (TDS) contained in the raw water, the control means 600 may collect the total dissolved solids (TDS) data of the raw water.

For reference, in the case of the detection means 500, the number of installed sensors may be adjusted according to importance. When selecting a sensor, the first turbidity sensor 510, which senses the turbidity of purified water, is selected as the top priority, and after that, the first TDS sensor 530, which senses the TDS amount of purified water, the second turbidity sensor, which senses the turbidity of raw water 520, and the second TDS sensor 540, which senses the TDS amount of raw water, have priority.

The control means 600 may perform a feedback operation based on water quality data measured by the detection means 500.

The detection means 500 may measure water quality, such as turbidity, and output it to the control means 600. The control means 600 may perform a feedback operation in response to the received raw water and/or purified water quality measurement data. Alternatively, the control means 600 may control other components to perform appropriate feedback operations based on the received raw water and/or purified water quality measurement data.

The control means 600 can provide the user with a real-time value of turbidity, whether the turbidity is within a normal range, notification of filter replacement, or the like, based on information such as the turbidity of raw water and/or purified water.

In detail, the detection means 500 measures the quality of raw water or purified water

The control means 600 performs feedback control based on water quality data measured by the detection means 500.

For example, when measuring the quality of raw water or purified water by the detection means 500, if the water quality data of the determined raw water or purified water is outside the preset normal range, the control means 600 may control to display information on the water quality abnormality of the raw water or purified water.

According to an embodiment of the present disclosure, the user can receive information on abnormal water quality of raw water and information on abnormal water quality of purified water separately, so that when there is an abnormality in purified water, the user can more quickly determine where the problem is.

In the case of the present disclosure, if the water quality data of the raw water or purified water is outside a preset normal range, it may further include an output part 800 that displays water quality abnormality information of the raw water or purified water.

The water dispensing apparatus may further include an output part 800 and/or an operation part.

The operation part may be configured to receive user input and may include one or more buttons. For example, the operation part may include a capacity button which is provided with a touch panel and selects water discharge capacity, a hot water button which selects hot water and further select the temperature of the hot water to be discharged, a purified water button which selects purified water, and a cold water button which selects cold water, and other function buttons.

The output part 800 may be equipped with a display device such as a display (not illustrated) or a light emitting diode (LED) (not illustrated). For example, the output part 800 may display information such as the operation status of the water dispensing apparatus, operating status related to error occurrence or the like, or water pollution level.

The output part 800 may be equipped with an audio device such as a speaker (not illustrated) and a buzzer (not illustrated). For example, the output part 800 may output a sound effect for the operating state of the water dispensing apparatus and output a predetermined warning sound when an error occurs.

The detection means 500 detects the turbidity and TDS amount of raw water or purified water, and even if the detected TDS amount is within the normal range, if the detected turbidity is outside the normal range, information on abnormal water quality of the raw water or purified water may be displayed on the output part 800.

The control means 600 may be connected to each component provided in the water dispensing apparatus. For example, the control means 600 may transmit and/or receive signals between each component provided in the water dispensing apparatus and control the overall operation of each component.

The control means 600 may include at least one processor, and may control the overall operation of the water dispensing apparatus using the processor included therein. Here, the processor may be a general processor such as a central processing unit (CPU). Of course, the processor may be a dedicated device such as an ASIC or another hardware-based processor.

The control means 600 may perform various operations based on data received through the detection means 500 including various sensors such as turbidity sensors 510 and 520. Additionally, the control means 600 may store data received through the detection means 500 in a memory (not illustrated).

Referring again to FIGS. 4 to 6, in the purified water flow path 220, a water outlet valve 770 is disposed between the filter 300 and the water outlet part 400 to regulate the flow of purified water flowing toward the water outlet 400, and the detection means 500 may be installed in the purified water flow path 220 connecting between the filter 300 and the water outlet valve 770.

As an example, the first turbidity sensor 510 may be installed in the purified water flow path 220 connecting between the filter 300 and the water outlet valve 770.

As another example, the first turbidity sensor 510 and the first TDS sensor 530 may be installed in the purified water flow path 220 connecting between the filter 300 and the water outlet valve 770.

In addition, an adjustment means 710 disposed between the water supply source and the filter 300 to adjust to be lower at least one of the pressure, flow velocity, or flow rate of the raw water flowing into the filter 300 may be installed in the raw water flow path 210, and the detection means 500 may be installed in a raw water flow path 210 connecting the adjustment means 710 and the filter.

As an example, the second turbidity sensor 520 may be installed in the raw water flow path 210 connecting the adjustment means 710 and the filter.

As another example, the second turbidity sensor 520 and the second TDS sensor 540 may be installed in the raw water flow path 210 connecting the adjustment means 710 and the filter.

In addition, the adjustment means 710 is configured to stabilize the flow of raw water flowing toward the second turbidity sensor 520, and may be provided as a pressure reducing valve.

The adjustment means 710 may be provided as an electronic valve that controls the inflow of raw water flowing toward the second turbidity sensor 520.

As described above, the reason for installing a pressure reducing valve or electronic valve in the front end of the second turbidity sensor 520 is because the water pressure of raw water is different according to the area where the water purifier is installed or the characteristics of the building, and the pressure reducing valve or electronic valve constantly and stably adjusts the water pressure, velocity, or flow of the raw water flowing into the second turbidity sensor 520, and thus the sensing value in the second turbidity sensor 520 does not fluctuate, and the turbidity of the raw water may be accurately sensed.

The water dispenser device according to the present disclosure can discharge purified water, cold water, and hot water.

However, the scope of the present disclosure is not limited thereto. The water dispensing apparatus of the present disclosure may be equipped with only one function selected from discharge of purified water, discharge of hot water, and discharge of cold water. Additionally, the water dispensing apparatus of the present disclosure may be equipped with at least two functions selected from the discharge of purified water, the discharge of hot water, and the discharge of cold water.

Referring to FIGS. 4 to 6, water supplied from the water supply source is guided to the filter 300 through the water supply flow path 210.

In addition, the purified water that has passed through the filter 300 flows through the purified water flow path 220.

One side of the purified water flow path 220 is connected to the outlet side of the filter 300, and the other side thereof is connected to the inlet side of the water outlet part 400, so that the water that has passed through the filter 300 is guided to the water outlet part 400.

The purified water in the purified water flow path 220 may be directly supplied to the water outlet part 400 in a purified state without heating or cooling.

Purified water in the purified water flow path 220 may be heated into hot water while passing through the hot water module 170 and may be supplied to the water outlet part 400 through the hot water flow path 240.

The hot water flow path 240 may be connected to the water outlet part 400 through a separate water outlet flow path 260. Accordingly, water that has passed through the hot water module 170 and the hot water flow path 240 may pass through the water outlet flow path 260 and then be discharged to the water outlet part 400.

In the case of the present disclosure, hot water and purified water may be discharged through one flow path.

However, the scope of the present disclosure is not limited thereto. Unlike the drawings, the water dispensing apparatus of the present disclosure may be formed with separate hot water flow paths and purified water flow paths.

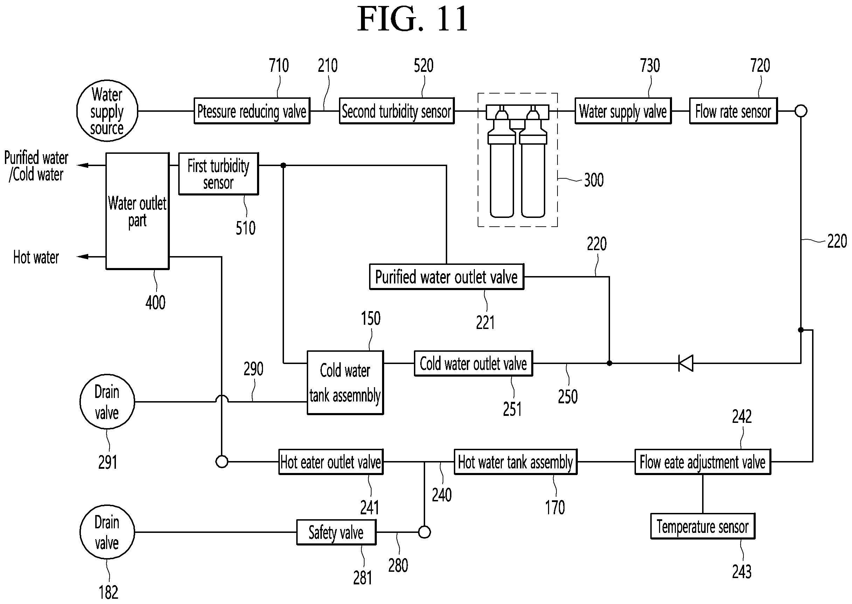

In this case, the purified water flow path 220 is directly connected to the water outlet valve 770, and the hot water flow path and the cold water flow path may be branched from the purified water flow path (see FIG. 11).

In other words, purified water may flow directly to the water outlet valve 770 without passing through the hot water module and hot water flow path.

When hot water is discharged, power is supplied to the hot water module 170, and when purified water is heated in the hot water module 170, hot water is discharged to the water outlet part 400.

When the power to the hot water module 170 is cut off, the purified water passing through the hot water module 170 is not heated and is discharged to the water outlet part 400 in a purified state.

In the case of hot water and purified water, both pass through the water supply flow path 210, the filter 300, the purified water flow path 220, the hot water module 170, the hot water flow path 240, and the water outlet flow path 260 and are discharged to the water outlet part 400.

The difference is whether the hot water module 170 operates. When discharging purified water, the control means switches the hot water module 170 to the off state so that the hot water module 170 does not operate, and the purified water that has passed through the hot water module 170 flows into the water outlet flow path 260 in a purified state. On the other hand, when hot water is discharged, the control means switches the hot water module 170 to the on state, so that the hot water module 170 operates, and the purified water passing through the hot water module 170 is heated as hot water.

The hot water module 170 includes a hot water tank. In addition, a heating flow path 171 through which purified water passing through the filter 300 passes is formed in the hot water tank, and when hot water is discharged, the purified water passing through the heating flow path 171 is heated to hot water. On the other hand, when purified water is discharged, the purified water that passes through the heating flow path 171 maintains the purified state without temperature change.

The water dispensing apparatus according to the present disclosure is, after branching from the first point P1 of the water supply flow path 210, may further include may further include an auxiliary flow path 230 joining the second point P2 of the water supply flow path 210 located at a rear end of the first point P1 based on the direction of water flow. Additionally, a flow rate adjustment valve 740 may be installed in the auxiliary flow path 230. The flow rate adjustment valve 740 may adjust the flow rate of water passing through the auxiliary flow path 230.

As an example, the flow rate adjustment valve 740 may adjust the flow rate of water passing through the auxiliary flow path 230 to a low level.

The control means is connected to the flow rate adjustment valve 740 and controls the flow rate adjustment valve 740. In detail, the control means may open or block the flow rate adjustment valve 740. In addition, the control means may adjust the degree of opening of the flow rate adjustment valve 740, thereby adjusting the flow rate of water passing through the auxiliary flow path 230.

A temperature sensor that detects the temperature of water passing through the auxiliary flow path 230 may be installed in the auxiliary flow path 230.

The temperature sensor may be installed integrally with the flow rate adjustment valve 740.

For reference, the temperature sensor may be installed in at least one of various water supply flow paths 210, purified water flow paths 220, hot water flow paths 240, and water outlet flow paths 260. Additionally, the temperature sensor may be installed in the hot water module 170 or in the water outlet valve 770, which will be described later.

Additionally, the control means is connected to a temperature sensor and may receive information on the temperature of water detected by the temperature sensor. Additionally, the output of the hot water module 170 or the auxiliary heater 750, which will be described later, may be controlled using the input temperature information.

A water supply valve 730 may be installed at the first point P1.

The water supply valve 730 may control the flow of water from the water supply flow path 210 to the auxiliary flow path 230.

In addition, the control means may control the operation of the water supply valve 730.

As an example, the water supply valve 730 may have one inlet and two outlets. Additionally, the inlet of the water supply valve 730 may be connected to the water supply flow path 210, one of the two outlets may be connected back to the water supply flow path 210, and the other may be connected to the auxiliary flow path 230.

The control means may open and close the inlet of the water supply valve 730 and open and close the outlet of the water supply valve 730. The control means may select and open one of the outlets of the water supply valve 730 and block the other one. The control means may open all the outlets of the water supply valve 730 and may also block all the outlets of the water supply valve 730.

Accordingly, the water flowing into the water supply valve 730 may be delivered to the water supply flow path 210 or the auxiliary flow path 230.

The water supply flow path 210 may include a first water supply flow path 211 that guides water toward the water supply valve 730 with respect to the water supply valve 730, and a second water supply flow path 212 that guides for directing water discharged from the water supply valve 730 to a filter 300.

For example, water flowing into the water supply valve 730 through the first water supply flow path 211 may not flow in the direction of the auxiliary flow path 230, but may only flow into the second water supply flow path 212.

As another example, water flowing into the water supply valve 730 through the first water supply flow path 211 may flow only in the direction of the auxiliary flow path 230 and may not flow into the second water supply flow path 212.

As another example, the water flowing into the water supply valve 730 through the first water supply flow path 211 partially flows in the direction of the auxiliary flow path 230, and partially flows in the direction of the second water supply flow path 212.

When hot water is discharged, the control means may control the water supply valve 730 so that the water flowing in through the first water supply flow path 211 passes through the auxiliary flow path 230. At this time, the water flowing into the water supply valve 730 is not discharged toward the second water supply flow path 212, but is discharged only toward the auxiliary flow path 230.

The water that has passed through the auxiliary flow path 230 may join the second water supply flow path 212 at the second point P2 and then be supplied to the filter 300.

Additionally, when purified water is discharged, the control means may control the water supply valve 730 so that water flowing in through the first water supply flow path 211 flows only through the second water supply flow path 212.

At this time, the water flowing into the water supply valve 730 is not discharged toward the auxiliary flow path 230, but is discharged only toward the second water supply flow path 212.

In addition, the water discharged to the second water supply flow path 212 may be supplied to the filter 300.

In general, purified water passes through the filter 300 and is then supplied to the water outlet part 400 through a flow path. Therefore, there is no need to adjust the flow rate of water passing through the flow path, and the larger the flow rate, the better.

However, in the case of hot water, after passing through the filter 300, it is heated into hot water and then supplied to the water outlet part 400. Therefore, in order to increase the hot water temperature, it is necessary to adjust the flow rate of water flowing into the hot water module 170 to be lower than that of purified water.

In this situation, when a flow rate adjustment valve is installed in the water supply flow path 210 that supplies water to the filter 300 and the flow rate supplied to the filter 300 is adjusted to a low level, the hot water temperature increases, thereby satisfying the hot water conditions. However, even when discharging purified water, a problem occurs in which the flow rate of purified water is reduced due to the low-adjusted flow rate.

To solve this problem, in the present disclosure, when purified water is discharged, the water flowing in through the first water supply flow path 211 is supplied to the filter 300 through the second water supply flow path 212. Since the flow rate adjustment valve is not installed in the second water supply flow path 212, a situation in which the purified water flow rate is lowered is prevented, and the purified water flow rate condition can be satisfied.

On the other hand, when hot water is discharged, the water flowing in through the first water supply flow path 211 is supplied to the filter 300 through the auxiliary flow path 230. A flow rate adjustment valve 740 is installed in the auxiliary flow path 230, and the flow rate adjusted to a low level by the flow rate adjustment valve 740 flows into the filter 300, thereby lowering the flow rate supplied to the hot water module 170, the hot water temperature may be increased and the hot water temperature conditions may be satisfied.

In other words, in the case of the present disclosure, the path of water flowing from the water supply flow path 210 to the filter 300 is divided into two, so that water flows only through the water supply flow path 210 when purified water is discharged, so that the flow rate does not decrease, and when hot water is discharged, water from the water supply flow path 210 may flow into the auxiliary flow path 230 to lower the flow rate, thus satisfying the temperature conditions of hot water.

Meanwhile, as described above, purified water discharged from the filter 300 flows toward the hot water module 170 through the purified water flow path 220.

Then, the water flowing into the hot water module 170 is heated and discharged as hot water, or is not heated and discharged as purified water and flows through the hot water flow path 240.

The hot water or purified water flowing through the hot water flow path 240 flows through the water outlet flow path 260 and is then supplied to the outside of the water purifier through the water outlet part 400.

One side of the water outlet flow path 260 is connected to the hot water flow path 240 and the other side thereof is connected to the water outlet part 400.

A water outlet valve 770 that controls the flow of water flowing toward the water outlet part 400 may be installed in the water outlet flow path 260.

The control means may control the operation of the water outlet valve 770.

In detail, the control means may control the water outlet valve 770 to be opened when purified water, hot water, or cold water is discharged and may control the water outlet valve 770 to be blocked in a standby state in which water is not discharged.

Meanwhile, after hot water is discharged, the hot water module 170 is in a heated state, and the hot water flow path 240, the hot water module 170, and the water outlet flow path 260 are also filled with hot water.

In this situation, when a user discharges purified water, a problem occurs in which hot water in the hot water flow path 240 or the hot water module 170 is discharged through the water outlet part 400 before the purified water is discharged.

Therefore, after hot water is discharged and before purified water is discharged, it is necessary to drain the hot water (residual water) remaining in the hot water flow path 240, the hot water module 170, and the water outlet flow path 260.

For this purpose, the water purifier of the present disclosure is provided with a drain flow path 270.

Additionally, the water outlet valve 770 is provided as a three-way valve, and a drain flow path 770 may be connected to the water outlet valve 770.

In addition, the water flowing into the water outlet valve 770 may flow toward the water outlet part 400 or may flow into the drain flow path 770.

The water outlet valve 770 may have one inlet and two outlets. In addition, the inlet of the water outlet valve 770 is connected to the water outlet part channel 260 extending toward the hot water channel 240. In addition, one of the two outlets is connected again to the water outlet flow path 260 extending toward the water outlet part 400, and the other one is connected to the drain flow path 770.

The control means may open and close the inlet of the water outlet valve 770 and open and close the outlet of the water outlet valve 770. The control means may select and open one of the outlets of the water outlet valve 770 and block the other one. The control means may open all the outlets of the water outlet valve 770, and may also block all the outlets of the water outlet valve 770.

Accordingly, the water flowing into the water outlet valve 770 may be discharged into the water outlet flow path 260 extending toward the water outlet part 400 and discharged into the water outlet part 400, or may be discharged into the drain flow path 770.

For example, when hot water is discharged, the water outlet valve 770 may be opened to discharge water into the water outlet flow path 260 extending toward the water outlet part 400.

As another example, when the hot water discharge ends, the outlet connected to the drain flow path 770 of the water outlet valve 770 is opened, so that the remaining water in the hot water flow path 240, the water outlet flow path 260, or the hot water module 170 may be drained.

As described above, when draining proceeds after hot water is discharged, the hot water flow path 240, the water outlet flow path 260, or the hot water module 170 is filled with purified water or is empty.

Therefore, it is possible to prevent the problem of the temperature of the purified water rising due to high-temperature residual water when discharging purified water after discharging hot water.

If purified water is discharged without draining after hot water is discharged, even if the hot water module 170 is turned off, there is a high temperature in the hot water flow path 240, since high-temperature residual water remains in the hot water flow path 240, the water outlet flow path 260, or the hot water module 170, a problem occurs in which high-temperature water is discharged through the water outlet part 400.

For reference, the drain may proceed until the remaining water in the hot water tank is discharged.

In the case of the present disclosure, when hot water discharge ends, draining may proceed regardless of whether purified water is dispensed.

In other words, when hot water is discharged, the hot water module 170 operates, and the outlet of the water outlet valve 770 on the side of the water outlet part 400 is opened.

Then, when the hot water discharge ends, the hot water module 170 stops operating, and the outlet of the water outlet valve 770 on the drain flow path 270 is opened, so that the hot water in the hot water flow path 240 or the hot water module 170 is drained through the drain flow path 270.

In addition, when the preset drain time elapses, the outlet of the water outlet valve 770 on the side of the drain flow path 270 is blocked.

In the case of the present disclosure, when hot water discharge ends and purified water is discharged, the elapsed time may be checked after hot water is discharged, and draining may be selectively performed according to the elapsed time.

In the case of the present disclosure, when hot water discharge ends and purified water is discharged, the remaining water temperature in the hot water flow path 240, the water outlet flow path 260, the hot water module 170, or the water outlet valve 770 is checked, and draining may be performed selectively according to the checked temperature.

Additionally, the water purifier according to the present disclosure may be equipped with a cold water outlet function.

For this purpose, the water purifier according to the present disclosure may further include a cold water flow path 250 having one side which branches off from the purified water flow path 220 and the other side which joins the water outlet flow path 260, and a cold water module 150 installed in the cold water flow path 250 and cooling the purified water passing the cold water flow path 250 with cold water.

In the purified water flow path 220, a cold and hot water valve 760 may be installed at a branch point of the cold water flow path 250.

The control means controls the operation of the cold and hot water valve 760.

For example, when discharging hot water or purified water, the control means may control the cold and hot water valve 760 to be opened toward the hot water module 170.

As another example, when cold water is discharged, the control means may control the cold and hot water valve 760 to be opened toward the cold water flow path 250.

The cold and hot water valve 760 may have one inlet and two outlets. In addition, the inlet of the cold and hot water valve 760 is connected to the purified water flow path 220 extending toward the filter 300. In addition, one of the two outlets is connected to the hot water module 170, and the other thereof is connected to the cold water flow path 250.

The control means may open and close the inlet of the cold and hot water valve 760, and open and close the outlet of the cold and hot water valve 760. The control means may select and open one of the outlets of the cold and hot water valve 760 and block the other one. The control means may open all the outlets of the cold and hot water valve 760 and may block all the outlets of the water outlet valve 770.

Accordingly, the water flowing into the cold and hot water valve 760 may be discharged into the purified water flow path 220 extending toward the hot water module 170 and thus may be supplied to the hot water module 170 or may be discharged into the cold water flow path 250.

As a modified example, after hot water is discharged, it is in a heated state, and the hot water flow path 240, and the hot water module 170, and the water discharge flow path 260 are also in a state of being filled with hot water.

In this situation, when a user discharges purified water, a problem occurs in which hot water in the hot water flow path 240 or the hot water module 170 is discharged through the water outlet part 400 before the purified water is discharged.

The control means may control the temperature of water discharged from the water outlet part 400 through cold water to a low level.

In this embodiment, if purified water is discharged within a preset reference time after hot water is discharged, the control means controls to open the outlet on the side of the purified water flow path 220 through which the cold and hot water valve 760 is connected to the hot water module 170 and to open the outlet on the side of the cold water flow path 250.

Here, the 'reference time' may be set in various ways. As an example, the 'reference time' may be set to 60 minutes.

After hot water is discharged, but before the reference time has elapsed, when purified water is discharged, the control means controls to open both outlets of the cold and hot water valve 760 simultaneously, and to open the outlet on the side of the cold water flow path 250 for the first time and then block the outlet thereon and to open the outlet on the side of the purified water flow path 220 connected to the hot water module 170 for the second time longer than the first time.

Here, 'first time' and 'second time' may be set in various ways. As an example, the 'first time' may be set to 3 seconds.

Additionally, the water discharged through the cold water flow path 250 and the hot water flow path 240 may merge in the water outlet flow path 260 and then be discharged through the water outlet part 400.

In the case of the present disclosure, the elapsed time is checked when hot water discharge ends, purified water is discharged, and after hot water is discharged, and then a portion of the cold water may be selectively added to the remaining water according to the elapsed time.

In the case of the present disclosure, the remaining water temperature in the hot water flow path 240 or the water outlet flow path 260, the hot water module 170, or the water outlet valve 770 is checked when hot water discharge ends and purified water is discharged, and then a portion of the cold water may be selectively added to the remaining water according to the checked temperature.

According to this, when discharging purified water through the hot water flow path, the problem of hot or lukewarm water being initially discharged when purified water is discharged due to residual hot water may be solved.

At least one of a pressure reducing valve 710 that adjusts the water pressure of water flowing in from the water supply source or a flow rate sensor 720 that checks the flow rate of water passing through the water supply flow path 210 may be installed in the water supply flow path 210.

For example, in the first water supply flow path 211, a pressure reducing valve 710 and a flow rate sensor 720 may be installed in order based on the water flow direction.

In addition, the control means is connected to the flow rate sensor 720 and may receive flow rate information detected by the flow rate sensor 720. Additionally, the output of the hot water module 170 or the auxiliary heater 750, which will be described later, may be controlled using the input flow rate information.

The water purifier according to the present disclosure has a hot water sterilization function.

An auxiliary heater 750 may be installed in the auxiliary flow path 230 to heat water flowing through the auxiliary flow path 230.

The auxiliary heater 750 may be formed behind the flow rate adjustment valve 740 based on the water flow direction.

The control means controls the overall operation of the auxiliary heater 750.

During hot water sterilization, the control means operates the auxiliary heater 750, and the hot water module 170 and cold water module 150 stop operating.

Then, the hot water heated while passing through the auxiliary heater 750 passes through the filter 300 and then flows toward the water outlet part 400.

As described above, when the auxiliary heater 750 operates, the auxiliary flow path 230 in which the auxiliary heater 750 is installed, the filter 300, the purified water flow path 220, the hot water flow path 240, the cold water flow path 250, and the water outlet flow path 260 may be sterilized with hot water.

The control means controls the water supply valve 730 so that water from the first water supply flow path 211 flows into the auxiliary flow path 230 during hot water sterilization.

The control means may control the cold and hot water valve 760 so that the water flowing through the purified water flow path 220 after being discharged from the filter 300 flows toward the cold water flow path 250 or the hot water flow path 240.

As an example, the control means may control the cold and hot water valve 760 so that water flowing through the purified water flow path 220 after being discharged from the filter 300 flows toward the cold water flow path 250.

As another example, the control means may control the cold and hot water valve 760 so that water flowing through the purified water flow path 220 after being discharged from the filter 300 flows sideways toward the hot water module 170 and the hot water flow path 240.

As another example, the control means may control the cold and hot water valve 760 so that water flowing through the purified water flow path 220 after being discharged from the filter 300 flows toward the cold water flow path 250 and the hot water flow path 240.

At this time, water flowing through the purified water flow path 220 after being discharged from the filter 300 may simultaneously flow toward the cold water flow path 250 and the hot water flow path 240.

The water flowing through the purified water flow path 220 after being discharged from the filter 300 sequentially may flow first toward the cold water flow path 250 and then flow toward the hot water flow path 240, or may flow first toward the hot water flow path 240 and then flow toward the cold water flow path 250.

The control means may control the water outlet valve 770 so that water in the water outlet flow path 260 flows toward the water outlet part 400 or the drain flow path 270 during hot water sterilization.

As an example, the control means may control the water outlet valve 770 so that water flowing into the water outlet valve 770 through the water outlet flow path 260 flows toward the water outlet part 400.

As another example, the control means may control the water outlet valve 770 so that water flowing into the water outlet valve 770 through the water discharge passage 260 flows toward the drain flow path 270.

As another example, the control means may control the water outlet valve 770 so that the water flowing into the water outlet valve 770 through the water outlet flow path 260 flows toward the water outlet part 400 and also flows toward the drain flow path 270.

At this time, water flowing into the water outlet valve 770 through the water outlet flow path 260 may simultaneously flow toward the water outlet part 400 and the drain flow path 270.

The water flowing into the water outlet valve 770 through the water outlet flow path 260 sequentially flows first toward the water outlet part 400 and then toward the drain flow path 270, or first flows toward the drain flow path 270 and then toward the water outlet part 400.

The water purifier 10 of the present disclosure may further include a steam flow path 280 for discharging steam generated when heating hot water in the hot water module 170 and a safety valve 190. Accordingly, it is possible to prevent the pressure inside the hot water tank included in the hot water module 170 from being excessively increased by steam. The safety valve 190 is configured to open at a set pressure, and may have various structures within a range that allows smooth discharge of steam inside the hot water tank.

Meanwhile, the steam flow path 280 may also be connected to the drain flow path 270. Accordingly, vapor discharged from the hot water tank may also be discharged to the outside of the water purifier 10 through the drain flow path 270.

According to the present disclosure as described above, since hot water sterilization progresses as continuous water discharge progresses, there is also an advantage that foreign matter may be removed simultaneously while sterilization of the flow path and valve progresses depending on the flow rate.

According to the present disclosure, a part of the hot water generated during hot water sterilization and hot water washing of the entire flow path included in the water purifier is discharged through the water outlet nozzle, and the rest thereof is drained to the outside of the water purifier, and thus there are advantages of, while a large amount of hot water is discharged through the water outlet nozzle, preventing safety accidents such as burns and preventing users from having to process large amounts of hot water.

FIG. 11 is a water piping diagram illustrating a water dispensing apparatus according to another embodiment of the present disclosure.

Referring to FIG. 11, water supplied from the water supply source is guided to the filter 300 through the water supply flow path 210.

The purified water that has passed through the filter 300 flows through the purified water flow path 220.

A pressure reducing valve 710 is installed in the water supply flow path 210.

A second turbidity sensor 520 is installed in the water supply flow path connecting the pressure reducing valve 710 and the filter 300, so that the turbidity of the raw water that has passed through the pressure reducing valve 710 may be sensed.

Meanwhile, one side of the purified water flow path 220 is connected to the outlet side of the filter 300, and the other side thereof is connected to the inlet side of the water outlet part 400, so that the water that has passed through the filter 300 is guided toward the water outlet part 400.

The purified water in the purified water flow path 220 may be directly supplied to the water outlet part 400 in a purified state without heating or cooling

A purified water outlet valve 221 that controls the flow of purified water flowing toward the water outlet part 400 may be installed in the purified water flow path 220.

The purified water outlet valve 221 is opened when purified water is discharged
In addition, the purified water in the purified water flow path 220 flows into the hot water flow path 240 branched from the purified water flow path 220, passes through the hot water tank assembly 170, is heated to hot water, and then may be supplied to the water outlet part 400.

A flow rate adjustment valve 242 is installed in the hot water flow path 240 branched from the purified water flow path 220 to adjust the flow rate of purified water flowing into the hot water tank assembly 170.

A temperature sensor 243 that senses the temperature of purified water flowing into the hot water tank assembly 170 may be installed in the flow rate adjustment valve 242.

As described above, purified water whose flow rate is adjusted while passing through the flow rate adjustment valve 242 may be heated to hot water while passing through the hot water tank assembly 170 and then supplied to the water outlet part 400.

A hot water outlet valve 241 that controls the flow of hot water flowing toward the water outlet part 400 may be installed in the hot water flow path 240.

The hot water outlet valve 241 is opened when hot water is discharged.

The hot water tank assembly 170 or the hot water flow path 240 through which hot water discharged from the hot water tank assembly 170 flows is connected to a drain flow path 280 through which lukewarm water, steam, or the like are discharged, and a safety valve 281 and a drain valve 281 may be installed in the drain flow path 280.

Purified water in the purified water flow path 220 flows into the cold water flow path 250 branched from the purified water flow path 220, passes through the cold water tank assembly 150, is cooled with cold water, and then may be supplied to the water outlet part 400.

A cold water outlet valve 251 that controls the flow of cold water flowing toward the water outlet part 400 may be installed in the cold water flow path 250
The cold water outlet valve 251 is opened when purified water is discharged.

Additionally, the outlet end of the cold water flow path 250 extending toward the water outlet part 400 may be joined to the outlet end of the purified water flow path 220. In other words, cold water in the cold water flow path 250 does not flow directly into the water outlet part 400, but may flow into the water outlet part 400 through the outlet end of the purified water flow path 220.

The cold water outlet valve 251 may be disposed to control the flow of purified water flowing toward the cold water tank assembly 150.

A drain flow path 290 through which cooling water is drained is connected to the cold water tank assembly 150, and a drain valve 291 may be installed in the drain flow path 290.

In this embodiment, a first turbidity sensor 510 may be installed in at least one of the purified water flow path 220, the hot water flow path 240, and the cold water flow path 250 at a position adjacent to the water outlet part 400.

The first turbidity sensor 510 may sense the turbidity of purified water, hot water, or cold water flowing into the water outlet part 400.

Again, referring to FIGS. 1 to 3, the water outlet part 400 is separated from the housing 100 and installed in a position spaced apart from the housing 100, and thus the detection means 500 may be installed in the raw water flow path 210 or the purified water flow path 220 exposed to the outside of the house 100.

The control means 600 may be also installed on the raw water flow path 210 or the purified water flow path 220 exposed to the outside of the housing 100.

The raw water flow path 210 may refer to a raw water pipe or a raw water hose.

The purified water flow path 220 may refer to a purified water pipe or a purified water hose.

In other words, the detection means 500 may be separated from the housing 100 and thus disposed outside the housing 100

As an example, the water dispensing apparatus according to the present disclosure may be provided as an under-sink type water purifier.

The water outlet part 400 is exposed to the upper side of the sink, and the housing 100 containing the filter 300 may be installed in the storage space below the sink.

The detection means 500 may be installed on various flow paths connecting the water outlet part 400 and the housing 100, and may be placed in the lower space of the sink.

The water outlet part 400 is separated from the housing 100 and installed in a position spaced apart from the housing 100, and the detection means 500 may be installed on the raw water flow path 210 or the purified water flow path 220 contained inside the housing 100.

The control means 600 may be also installed on the raw water flow path 210 or the purified water flow path 220 contained inside the housing 100.

In a state where the water outlet part 400 is exposed to the upper side of the sink and the housing 100 containing the filter 300 is installed in the storage space below the sink, the detection means 500 may be contained in the inside of the housing 100.

The detection means 500 may be coupled to the housing 100 to be exposed to the outside of the housing 100.

The water outlet part 400 may be integrally provided with the housing 100 to be exposed to the outside of the housing 100, and the detection means 500 may be connected to the housing 100 and the water outlet part 400 and be installed on the raw water flow path 210 or the purified water flow path 220 contained inside the housing 100.

The control means 600 may be also installed on the raw water flow path 210 or the purified water flow path 220 contained inside the housing 100.

In other words, the detection means 500 may be formed integrally with the housing 100.

As an example, the water dispensing apparatus according to the present disclosure may be equipped as a desktop-type water purifier.

The water outlet part 400 may be coupled to the housing 100 to be exposed to the outside of the housing 100.

The detection means 500 may be installed in the internal space of the housing 100.

The detection means 500 may be coupled to the housing 100 to be exposed to the outside of the housing 100.

Meanwhile, as described above, in order for the detection means 500 to be separated from the housing 100 and placed outside the housing 100, a separate cover is required.

Referring to FIGS. 1 to 3, the detection means 500 includes an outer cover 551, 552 forming an installation space 554, 555, 556 on the inside, and a first turbidity sensor (51) which senses the turbidity of the purified water.

The outer covers 551 and 552 have a first cover 551 that has one side open and may include a first cover 551 which forms an installation space 554, 555, and 556 on the inside, and a second cover 552 that covers one open side of the first cover 551.

A plurality of intermediate walls 553 and 557 may be formed inside the first cover 551 to partition the installation spaces 554, 555 and 556. The intermediate walls 553 and 557 may divide the installation spaces 554, 555, and 556 in a vertical direction or in a left and right direction, and the installation spaces 554, 555, and 556 may be divided into at least three spaces by the intermediate walls 553 and 557.

In this embodiment, the purified water flow path 220 may include a purified water input pipe 220a disposed below the first turbidity sensor 510 and supplies purified water supplied from the filter 300 to the first turbidity sensor 510, and a purified water output pipe 220b disposed above the first turbidity sensor 510 and supplying purified water that has passed through the first turbidity sensor 510 to the water outlet part 400.

Due to the above structure, purified water may pass through the first turbidity sensor 510 while flowing from the lower side to the upper side.

When purified water flows from the lower side to the upper side as described above, the flow rate of the purified water decreases and the flow becomes more stable, allowing the first turbidity sensor 510 to more accurately sense the turbidity of the purified water.

The detection means 500 further include a first TDS sensor 510 disposed between the first turbidity sensor 510 and the purified water output pipe 220b and measuring the amount of TDS of the purified water that has passed through the first turbidity sensor 510.

The purified water flow path 220 further includes a purified water connection pipe 220c connecting the outlet end of the first turbidity sensor 510 and the inlet end of the first TDS sensor 530.

Due to the above structure, purified water may pass through the first TDS sensor 530 while flowing from the lower side to the upper side.

When purified water flows from the lower side to the upper side as described above, the flow rate of the purified water decreases and the flow becomes more stable, allowing the first TDS sensor 530 to more accurately sense the amount of TDS contained in the purified water.

The detection means 500 further includes a second turbidity sensor 520 installed in the installation space 555 to sense the turbidity of raw water.

The raw water flow path 210 may include a raw water input pipe 210a disposed below the second turbidity sensor 520 and supplies raw water supplied from the water supply source to the second turbidity sensor 520 and a raw water output pipe 210b disposed above the second turbidity sensor 520 and supplying raw water that has passed through the second turbidity sensor 520 to the filter 300.

Due to the above structure, raw water may pass through the second turbidity sensor 520 while flowing from the lower side to the upper side.

When the raw water flows from the lower side to the upper side as described above, the flow rate of the raw water is lowered and the flow becomes more stable, allowing the second turbidity sensor 520 to more accurately sense the turbidity of the raw water.

An adjustment means 710 installed in the installation space 555, and disposed between the second turbidity sensor 520 and the raw water input pipe 210a so that the pressure of the raw water flowing into the raw water input pipe 210a is adjusted to be lowered and then supplies the pressure of the raw water to the second turbidity sensor 520 may be installed in the raw water flow path 210.

Due to the above structure, while raw water flows from the lower side to the upper side, the raw water may pass through the adjustment means 710 and the first TDS sensor 530 in order.

When the raw water flows from the lower side to the upper side and passes through the adjustment means 710 as described above, the flow rate of the raw water is lowered and the flow becomes more stable, allowing the second turbidity sensor 520 to more accurately sense the turbidity of the raw water.

The control means 600 may be installed in the installation space 554 and placed on the upper portion of the purified water output pipe 220b.

A temperature sensor 558, a humidity sensor, and various sensors capable of collecting data on the surrounding environment may be installed in the installation spaces 554, 555, and 556.

The control means 600 may be connected to a separate output part 800 and transmit the input water quality data to the output part 800.

The control means 600 is connected to a separate control system, and the control system is connected to a separate output part 800 to transmit water quality data input through the control means 600 to the output part 800.

The control system may transmit various notifications to the output part 800 based on water quality data input through the control means 600.

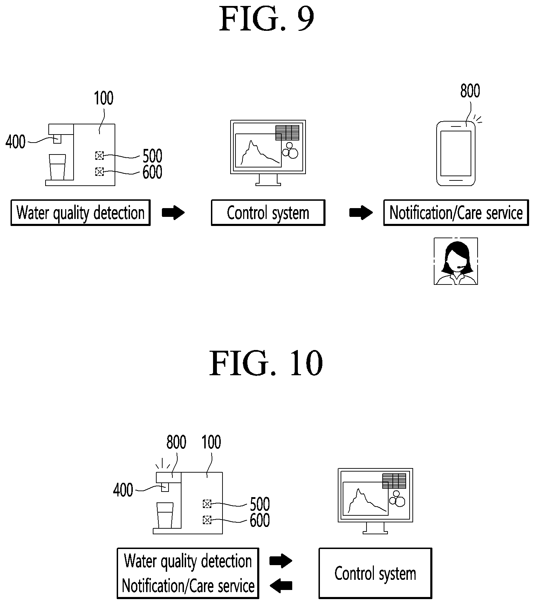

FIGS. 9 to 10 are diagrams conceptually illustrating a monitoring system for a water dispensing apparatus according to the present disclosure.

Referring to FIG. 9, the output part 800 is an external terminal such as a mobile phone, and may communicate with the control means 600 to output the water quality status.

Accordingly, the user can receive notifications related to water quality conditions through an application on a portable terminal or through an information phone call.

Referring to FIG. 10, the output part 800 is formed in the housing 100 or the water outlet part 400 and may output the water quality status visually or audibly.

According to the above, the detection means 500 of the present disclosure detects the turbidity or TDS amount of raw water or purified water.

As an example, the detection means 500 may detect the turbidity and TDS amount of purified water.

The turbidity and TDS amount of purified water measured in real time are output to the control means 600.

At this time, the control system that communicates with the control means 600 may monitor the turbidity and TDS amount of purified water in real time.

The control means 600 may include a communication module that performs wireless communication through Wi-Fi or the like.

The control system may be Server or Argus.

For reference, Argus is a system and network monitoring application. It is designed to monitor the state of network services, servers, and other network hardware, and has the function to send alerts when the Argus detects problems.

The control means 600 can independently output the turbidity and TDS amount of purified water in real time to the output part 800.

The control means 600 can independently output to the output part 800 whether the turbidity and TDS amount of the purified water in real time are within the suitable range for drinking.

In a control system that communicates with the control means 600, the turbidity and TDS amount of purified water in real time may be output to the output part 800.

The control system that communicates with the control means 600 may output to the output part 800 whether the turbidity and TDS amount of the purified water in real time are within the suitable range for drinking.

The control means 600 or the control system may display information on purified water quality abnormalities on the output part 800 even if the TDS amount of the real-time purified water detected by the detection means 500 is within the normal range, if the turbidity of the detected purified water is outside the preset suitable drinking range.

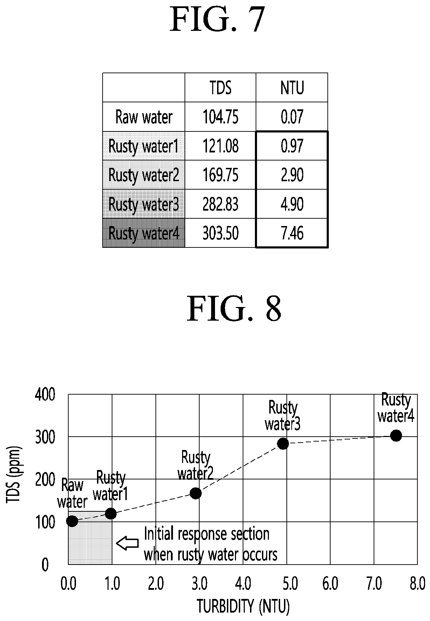

FIG. 7 is a table comparing the TDS amount and turbidity (NTU) of rusty water of various concentrations, and FIG. 8 is a graph comparing the amount of TDS and turbidity (NTU) of rusty water of various concentrations.

Referring to FIGS. 7 and 8, at the beginning of rusty water generation (rust 1), the TDS amount of purified water is within the normal range. If the suitability for drinking is determined based on the TDS amount of purified water, a situation may arise where the user drinks the rusty water without being aware of this.

In other words, it is difficult to determine whether purified water is suitable for drinking based on the TDS amount of the purified water.

In the case of the present disclosure, the real-time turbidity (NTU) of the purified water is sensed to determine whether it is suitable for drinking.

In detail, the control means 600 or the control system may determine that the purified water is suitable for drinking if the real-time turbidity (NTU) of the purified water is at the preset normal range or less, even if the TDS amount of the purified water detected in real time is within the normal range, determine that the purified water is unsuitable for drinking, if the real-time turbidity (NTU) of the purified water exceeds the preset normal range, and output to the output part 800 so that the user can recognize this situation.

The control means 600 or the control system may determine that the purified water is unsuitable for drinking even if the turbidity (NTU) of the purified water detected in real time is within the normal range, if the TDS amount of the purified water in real time exceeds the preset normal range, and output to the output part 800 so that the user can recognize this situation.

As described above, when sensing the real-time turbidity (NTU) of raw water or purified water, there is an advantage in being able to detect rust inflow early and respond proactively.

As described above, when the inflow of rusty water is detected by sensing the real-time turbidity (NTU) of raw water or purified water, the control means may perform control to close the water outlet valve to prevent water from being discharged from the water outlet part 400, or to close the water supply valve to block the inflow of raw water.

At this time, the output part 800 may output whether the purified water is suitable for drinking.

The output part 800 may output notifications such as 'suitable for drinking', 'unsuitable for drinking', 'good', 'replace filter', 'add filter', 'sterilize flow path', 'replace flow path', or the like.

For example, if the water quality data of raw water or purified water measured by the turbidity sensors 510 and 520 is outside a preset normal range, a filter replacement notification may be displayed on the output part 800.

As mentioned above, the real-time turbidity value of purified water may be used as an important indicator to evaluate the filtration performance of a filter. In addition, when determining filter performance, i.e., filter replacement time, based on the turbidity value sensed by the turbidity sensor, without being limited by the type of filter installed in the water dispensing apparatus (for example, reverse osmosis filter, hollow fiber membrane filter, or the like), filter performance may be determined.

For reference, the TDS amount of purified water measured by the TDS sensor may only determine the performance of the reverse osmosis filter, and it is difficult to determine the performance of the hollow fiber membrane filter.

In addition, the output part 800 may output values representing the turbidity of the purified water and the amount of TDS of the purified water.

The output part 800 may output a level indicating the turbidity of the purified water and the amount of TDS of the purified water.

The output part 800 may recommend the filter 300 based on the turbidity of the purified water and the TDS amount of the purified water.

Additionally, the output part 800 may display a customized filter based on water quality data measured by the detection means of the water dispensing apparatus.

The output part 800 may have an input function.

When the user inputs information on a specific water dispensing apparatus into the output part 800, the output part 800 may display a customized filter based on the water quality data of the specific water dispensing apparatus entered by the user..

In other words, the type of filter, number of filters, and combination of filters may be customized according to the water quality data of the water dispensing apparatus.

The water dispensing apparatus may be communicatively connected to the water quality control system, either wired or wirelessly.

When the user inputs geographical and environmental information on a specific area, location, building, or the like through the output part 800, the water quality control system may recommend customized water dispensing apparatus to the user from the data of the location input to the output part 800. The customized water dispensing apparatus may vary in the number or type of filters accommodated therein for each type.

In general, the water quality of water supply varies according to the area and building where the water dispensing apparatus is installed. Therefore, it is necessary to apply a water quality customized filter for each location where the water dispensing apparatus is installed, and the water dispensing apparatus according to the present disclosure may suggest and recommend a customized filter to the user.

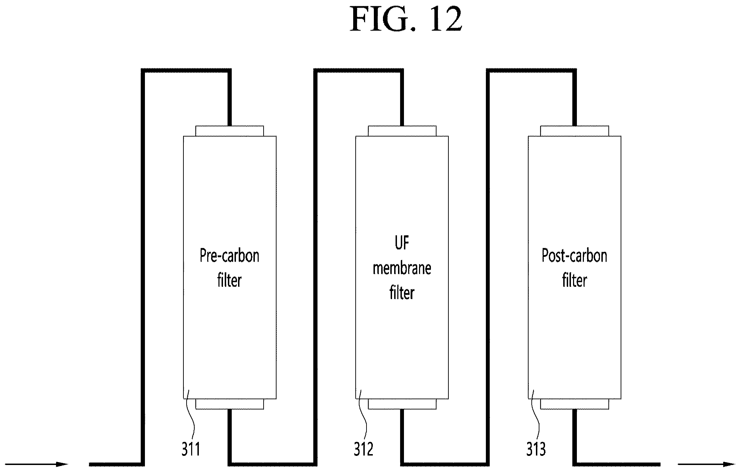

FIGS. 12 to 15 are diagrams illustrating various embodiments of filters applied to water dispensing of the present disclosure.

Referring. FIGS. 12 to 15, various filters 300 may be applied to the water dispensing apparatus of the present disclosure.

In other words, in the water dispensing apparatus of the present disclosure, a combination of various filters 300 may be recommended to the user according to the condition of the raw water at the installed location.

Furthermore, a water dispensing apparatus that accommodates a combination of various filters 300 according to the condition of raw water at the location where the water dispensing apparatus is to be installed may be recommended to the user.

As the filter 300, one filter may be provided or a plurality of filters may be provided.

The filter 300 may be configured to have a plurality of filter materials built into one filter housing.

In the description that follows, carbon blocks may be manufactured from different components. In addition, the carbon blocks may be manufactured with different composition ratios.

In the description described later, a membrane filter or a reverse osmosis filter may be applied instead of a hollow fiber membrane filter, and a membrane filter or reverse osmosis filter may be applied together with the hollow fiber membrane filter.

First, referring to FIG. 12, based on the water flow direction, the filter 300 may be composed of a pre-carbon filter 311 a built-in carbon block, a hollow fiber membrane filter 312 built-in a plurality of hollow fiber membranes, and a post-carbon filter 312 built-in a carbon block.

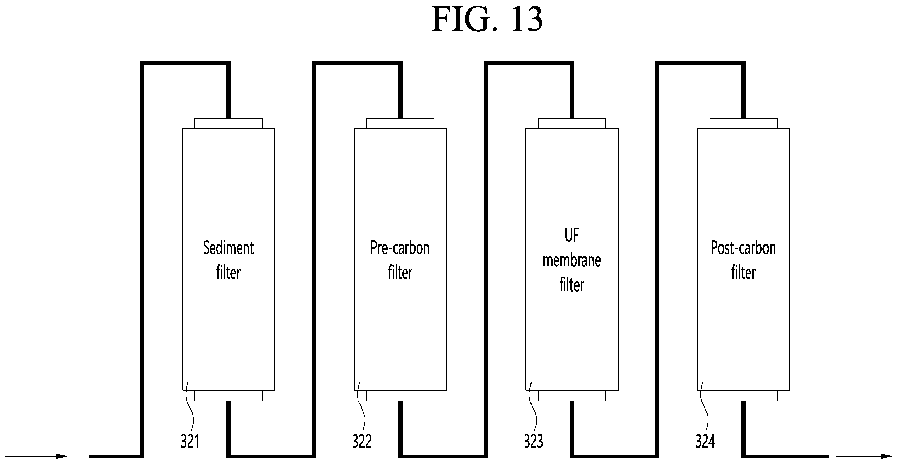

Referring to FIG. 13, based on the water flow direction, the filter 300 may be composed of a sediment filter 321, a free carbon filter 321 built-in a carbon block, a hollow fiber membrane filter 323 built-in a plurality of hollow fiber membranes, and a post-carbon filter 324 built-in a carbon block.

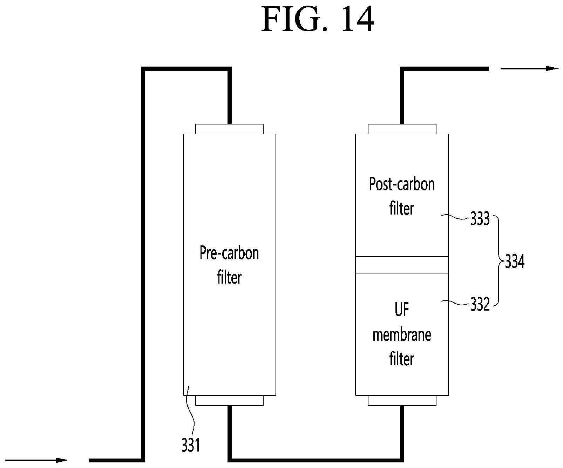

Referring to FIG. 14, based on the water flow direction, the filter 300 may be composed of a pre-carbon filter 331 built-in a carbon block and a composite filter 334.

The composite filter 334 may be constructed by embedding a plurality of hollow fiber membranes and carbon blocks in one filter housing.

In detail, a hollow fiber membrane filter 332 built in a plurality of hollow fiber membranes may be disposed at the lower end of the composite filter 334, and a post-carbon filter 312 built in a carbon block is disposed at the upper end of the composite filter 334.

Water flowing into the composite filter 334 may first pass through the hollow fiber membrane filter 332 and then pass through the post-carbon filter 312 before being discharged.

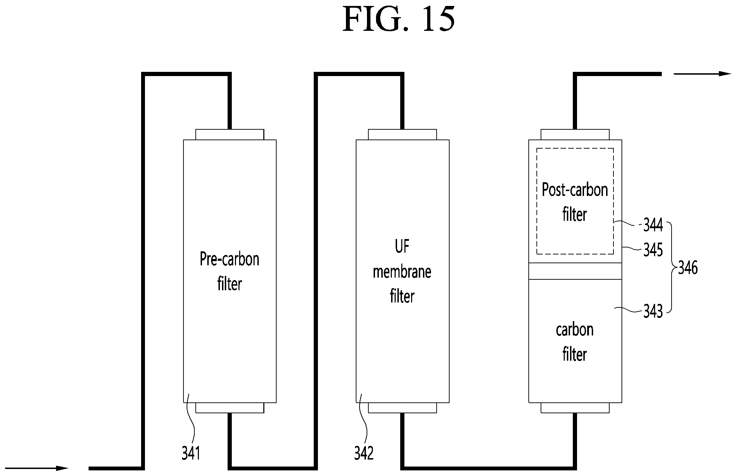

Referring to FIG. 15, based on the water flow direction, the filter 300 may be composed of a pre-carbon filter 341 built-in a carbon block, a hollow fiber membrane filter 342 built-in a plurality of hollow fiber membranes, and a composite filter 346.

The composite filter 346 may be constructed by embedding a plurality of carbon blocks in one filter housing.

In detail, a carbon filter 343 built-in a carbon block may be placed at the lower end of the composite filter 346, and a post carbon filter 344 built-in a carbon block may also be placed on the upper end of the composite filter 346.

Water flowing into the composite filter 346 may first pass through the carbon filter 343 and then pass through the post carbon filter 344 before being discharged.

Various types of known filters may be selected individually or in various combinations and applied to the water dispensing apparatus of the present disclosure.

According to the present disclosure as described above, there may be an advantage of sensing the turbidity of raw water or purified water in real time, so that if the quality of raw water deteriorates due to the inflow of rusty water or the like, it can be detected early and proactively respond.

Above all, there may be also advantages of actually sensing the turbidity of the water supplied to the user, notifying the user in real time when the water quality decreases and becomes unsuitable for drinking, and further blocking water discharge to prevent the user from drinking water that is unsuitable for drinking.

There may be an advantage of being able to monitor water quality more accurately by measuring and analyzing at the final stage of the water quality of the raw water flowing in from water supply sources including water supply pipes and the water discharged through the water outlet nozzle and to provide appropriate services.

In addition, there may be an advantage in that sensors are miniaturized that may measure turbidity which is one of the criteria for determining whether the water is suitable for drinking and TDS levels which are commonly used as water quality characteristics, and sensors with improved sensing precision are applied to measure the quality of raw water and purified water more quickly and accurately.

In addition, there may be an advantage that it can be easily applied to products that are already installed or have been commercialized.

Additionally, there may be an advantage of being able to manage and visualize differences in water quality measurements between raw water and purified water.

Additionally, there may be an advantage of modularizing a plurality of sensors to reduce material costs and create a compact product.

Additionally, there may be the advantage of being able to easily determine and manage the pollution level of raw water and purified water.

In the above, preferred embodiments of the present disclosure have been illustrated and described, but the present disclosure is not limited to the specific embodiments described above, of course, various modified embodiments may be made by those skilled in the art without departing from the gist of the disclosure as claimed in the patent claims, and these modified embodiments should not be understood individually from the technical idea or perspective of the present disclosure.

## Claims

1. A water dispensing apparatus comprising:
a filter (300) configured to generate purified water by filtering water;
a water supply flow path (210) for supplying water from an external water supply source to the filter (300);
a purified water flow path (220) for supplying the purified water toward a water outlet part (400);
a detection means (500) installed in at least one of the water supply flow path (210) and the purified water flow path (220) and configured to measure water quality data; and
a control means (600) configured to receive the water quality data detected by the detection means (500).

2. The water dispensing apparatus of claim 1,
wherein the detection means (500) includes a turbidity sensor (510, 520) configured to irradiate light to a portion of the water and to sense turbidity of the water based on a pattern of scattered light received, wherein the water quality data includes the turbidity.

3. The water dispensing apparatus of claim 2,
wherein the turbidity sensor (510, 520) includes a measuring container with a light incident part to input laser light therethrough, a light scattering space formed inside the measuring container, and a light emitting part to receive the pattern of scattered light generated in the light scattering space for measuring microscopic turbidity or pollution by bacteria or microorganisms.

4. The water dispensing apparatus of claim 3,
wherein the measuring container has a cylindrical shape and/or is made of at least one of metal, glass, synthetic resin, and combinations thereof and/or has scattering protrusions or scattering layers formed on an inner surface thereof.

5. The water dispensing apparatus according to any one of the preceding claims,
wherein the detection means (500) includes at least one of an ion sensor, a chlorine sensor, a Total Dissolved Solids (TDS) sensor, and a Biochemical Oxygen Demand (BOD) sensor; and/or
wherein the detection means (500) is configured to measure at least one of pH, residual chlorine, Total Dissolved Solids (TDS), and dissolved oxygen of the water; the water quality data including the at least one of the residual chlorine, the Total Dissolved Solids (TDS), and the dissolved oxygen.

6. The water dispensing apparatus of according to any one of the preceding claims,
wherein the detection means (500) includes a first turbidity sensor (510) installed in the purified water flow path (220) to sense turbidity of the purified water; and/or
wherein the detection means (500) includes a first TDS sensor (530) installed in the purified water flow path (220) to sense the amount of total dissolved solids (TDS) contained in the purified water.

7. The water dispensing apparatus of according to any one of claims 1 to 5,
wherein the detection means (500) includes a first turbidity sensor (510) and a first TDS sensor (530), and
the first turbidity sensor (510) is disposed between the filter (300) and the water outlet part (400) and the first TDS sensor (530) is disposed between the first turbidity sensor (510) and the water outlet part (400).

8. The water dispensing apparatus according to any one of the preceding claims,
wherein the detection means (500) includes a second turbidity sensor (520) installed in the water supply flow path (210) to sense turbidity of the water flowing through the water supply flow path (210); and/or
wherein the detection means (500) includes a second TDS sensor (540) installed in the water supply flow path (210) to sense the amount of total dissolved solids (TDS) contained in the water flowing through the water supply flow path (210).

9. The water dispensing apparatus of according to any one of claims 1 to 7,
wherein the detection means (500) includes a second turbidity sensor (520) and a second TDS sensor (540), and
the second turbidity sensor (520) is disposed between the external water supply source and the filter (300) and the second TDS sensor (540) is disposed between the external water supply source and the second turbidity sensor (510).

10. The water dispensing apparatus according to any one of the preceding claims,
wherein the control means (600) is configured to perform feedback control based on the water quality data measured by the detection means (500); and/or
wherein the control means (600) is configured to control an output part to display information on abnormal water quality when the water quality data is outside a preset normal range.

11. The water dispensing apparatus according to any one of the preceding claims,
wherein a water outlet valve (770) is disposed between the filter (300) and the water outlet part (400) and configured to control the flow of purified water.

12. The water dispensing apparatus of claim 11,
wherein the detection means (500) is installed in the purified water flow path (220) between the filter (300) and the water outlet valve (770).

13. The water dispensing apparatus according to any one of the preceding claims,
wherein an adjustment means (710) is disposed between the external water supply source and the filter (300) and configured to adjust at least one of pressure, flow velocity and flow rate of the water flowing into the filter (300).

14. The water dispensing apparatus of claim 13,
wherein the detection means (500) is installed in the water supply flow path (210) between the adjustment means (710) and the filter (300).

15. The water dispensing apparatus according to any one of the preceding claims, further including a housing (100), in which the filter (300) is disposed;
wherein the detection means (500) is installed to be exposed to the outside of the housing (100) or accommodated inside the housing.
